(19) Europäisches Patentamt
European Patent Office
Office européen des brevets

(11) Veröffentlichungsnummer: **0 318 782 B1**

# (12) EUROPÄISCHE PATENTSCHRIFT

(45) Veröffentlichungstag der Patentschrift: **20.01.93**

(51) Int. Cl.5: **C07C 275/54**, C07C 335/26, C07C 331/28, C07C 265/12, A01N 47/34

(21) Anmeldenummer: **88119293.4**

(22) Anmeldetag: **21.11.88**

## (54) Substituierte Benzoyl(thio)harnstoffe.

(30) Priorität: **01.12.87 DE 3740633**

(43) Veröffentlichungstag der Anmeldung:
**07.06.89 Patentblatt 89/23**

(45) Bekanntmachung des Hinweises auf die Patenterteilung:
**20.01.93 Patentblatt 93/03**

(84) Benannte Vertragsstaaten:
**BE CH DE FR GB IT LI NL**

(56) Entgegenhaltungen:
**EP-A- 0 180 818**
**EP-A- 0 194 688**
**EP-A- 0 219 460**
**EP-A- 0 277 748**

(73) Patentinhaber: **BAYER AG**
**W-5090 Leverkusen 1 Bayerwerk(DE)**

(72) Erfinder: **Sirrenberg, Wilhelm, Dr.**
**Wuppertaler Strasse 21**
**W-4322 Sprockhövel(DE)**
Erfinder: **Marhold, Albrecht, Dr.**
**Carl-Duisberg-Strasse 329**
**W-4090 Leverkusen 1(DE)**
Erfinder: **Becker, Benedikt, Dr.**
**Metzkausener Strasse 14**
**W-4020 Mettmann(DE)**

EP 0 318 782 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Benzoyl(thio)harnstoffe, Verfahren und neue Zwischenprodukte zu ihrer Herstellung sowie ihre Verwendung als Schädlingsbekämpfungsmittel, insbesondere als Insektizide.

Es ist bereits bekannt, daß bestimmte Benzoylharnstoffe, wie z. B. 1-(2-Chlor-4-fluor-benzoyl)-3-(1,1,2,3,3,3-hexafluorpropoxyphenyl)-harnstoff, insektizide Eigenschaften aufweisen (vgl. DE-OS 32 17 619). In der prioritätsälteren, nicht vorveröffentlichten EP-A-0 277 748 werden die Verbindung N-2,6-Difluorbenzoyl-N'-[2-fluor-4-(1,1,2,2-tetrafluorethoxy)phenyl]-harnstoff und ihre insektiziden Eigenschaften beschrieben.

Es wurden nun die neuen substituierten Benzoyl(thio)harnstoffe der allgemeinen Formel (I),

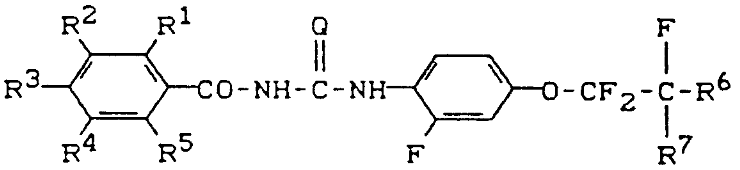

(I)

in welcher

Q für Sauerstoff oder Schwefel steht,
$R^1$ für Wasserstoff, Halogen, Nitro, Methyl oder Trifluormethyl steht,
$R^2$ für Wasserstoff, Fluor oder Chlor steht,
$R^3$ für Wasserstoff, Fluor oder Chlor steht,
$R^4$ für Wasserstoff oder Fluor steht,
$R^5$ für Wasserstoff oder Halogen steht,
$R^6$ für Wasserstoff, Chlor oder Fluor steht und
$R^7$ für Fluor, Chlor oder Trifluormethyl steht,

ausgenommen die Restekombination, in welcher $R^1$, $R^5$ und $R^7$ für Fluor, $R^2$, $R^3$, $R^4$ und $R^6$ für Wasserstoff und Q für Sauerstoff stehen,
gefunden.

Die oben ausgenommene Restekombination ist auch bei den folgenden allgemeinen Formeln entsprechend ausgenommen.

Halogen bedeutet in den obigen Definitionen Fluor, Chlor, Brom und Iod, vorzugsweise Fluor, Chlor und Brom, insbesondere Fluor und Chlor.

Weiter wurde gefunden, daß man die neuen substituierten Benzoyl(thio)harnstoffe der allgemeinen Formel (I) erhält, wenn man

(a) substituierte Aniline der allgemeinen Formel (II),

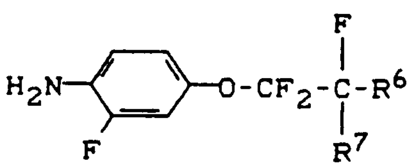

(II)

in welcher

$R^6$ und $R^7$ die oben angegebenen Bedeutungen haben,

mit Benzoyliso(thio)cyanaten der allgemeinen Formel (III)

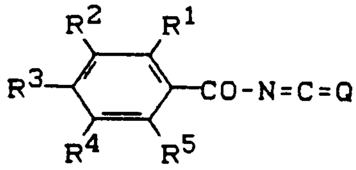

(III)

in welcher

Q, $R^1$, $R^2$, $R^3$, $R^4$ und $R^5$ die oben angegebenen Bedeutungen haben,

gegebenenfalls in Gegenwart eines Verdünnungsmittels umsetzt, oder

(b) substituierte Phenyliso(thio)cyanate der allgemeinen Formel (IV)

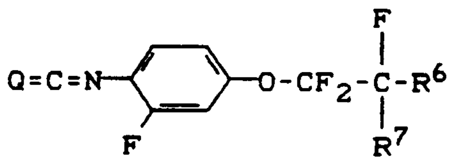

(IV)

in welcher

Q, $R^6$ und $R^7$ die oben angegebenen Bedeutungen haben,

mit Benzoesäureamiden der allgemeinen Formel (V)

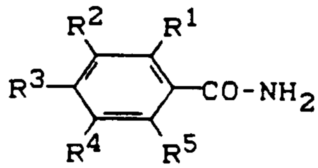

(V)

in welcher

$R^1$, $R^2$, $R^3$, $R^4$ und $R^5$ die oben angegebenen Bedeutungen haben,

gegebenenfalls in Gegenwart eines Katalysators und gegebenenfalls in Gegenwart eines Verdünnungsmittels umsetzt.

Die neuen Verbindungen der allgemeinen Formel (I) verfügen über Eigenschaften, die ihre Verwendung als Schädlingsbekämpfungsmittel ermöglichen; sie zeigen gute arthropodizide Eigenschaften und zeichnen sich insbesondere durch sehr starke insektizide Wirksamkeit aus.

Bevorzugt werden die neuen substituierten Benzoyl(thio)-harnstoffe der Formel (I), in welcher

Q für Sauerstoff oder Schwefel steht,

$R^1$ für Wasserstoff, Fluor, Chlor, Brom, Nitro, Methyl oder Trifluormethyl steht,

$R^2$ für Wasserstoff, Fluor oder Chlor steht,

$R^3$ für Wasserstoff, Fluor oder Chlor steht,

$R^4$ für Wasserstoff oder Fluor steht,

$R^5$ für Wasserstoff, Fluor oder Chlor steht,

$R^6$ für Wasserstoff, Chlor oder Fluor steht und

$R^7$ für Fluor, Chlor oder Trifluormethyl steht,

wobei die Restekombination ausgenommen ist, in welcher $R^1$, $R^5$ und $R^7$ für Fluor, $R^2$, $R^3$, $R^4$ und $R^6$ für Wasserstoff und Q für Sauerstoff stehen.

Besonders bevorzugt werden die neuen substituierten Benzoyl(thio)harnstoffe der Formel (I), in welcher

Q für Sauerstoff oder Schwefel steht,

$R^1$ für Fluor, Chlor oder Brom steht,

$R^2$ für Wasserstoff steht,

$R^3$ für Wasserstoff oder Fluor steht,

$R^4$ für Wasserstoff oder Fluor steht,

$R^5$ für Wasserstoff oder Fluor steht,

$R^6$ für Wasserstoff steht und

$R^7$ für Trifluormethyl steht.

Beispiele für Verbindungen der Formel (I) sind in der nachstehenden Tabelle 1 aufgeführt - vgl. auch die Herstellungsbeispiele.

Tabelle 1: Beispiele für die Verbindungen der Formel (I)

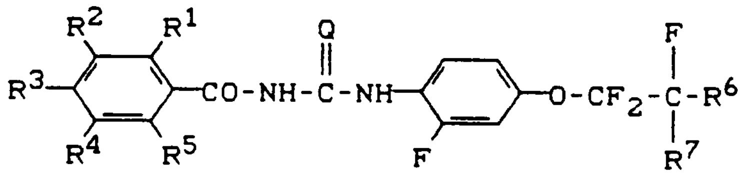
(I)

| Q | $R^1$ | $R^2$ | $R^3$ | $R^4$ | $R^5$ | $R^6$ | $R^7$ |
|---|---|---|---|---|---|---|---|
| O | F | H | H | H | F | H | Cl |
| S | F | H | H | H | F | H | Cl |
| O | Cl | H | H | H | H | H | Cl |
| S | Cl | H | H | H | H | H | Cl |
| O | Cl | H | H | H | F | H | Cl |
| S | Cl | H | H | H | F | H | Cl |
| O | Cl | H | F | H | H | H | Cl |
| S | Cl | H | F | H | H | H | Cl |
| O | Cl | H | H | F | H | H | Cl |
| S | Cl | H | H | F | H | H | Cl |
| O | F | H | H | H | H | H | Cl |
| S | F | H | H | H | H | H | Cl |

Tabelle 1 - Fortsetzung

| Q | $R^1$ | $R^2$ | $R^3$ | $R^4$ | $R^5$ | $R^6$ | $R^7$ |
|---|---|---|---|---|---|---|---|
| O | Br | H | H | H | H | H | Cl |
| S | Br | H | H | H | H | H | Cl |
| S | F | H | H | H | F | H | F |
| O | Cl | H | H | H | H | H | F |
| S | Cl | H | H | H | H | H | F |
| O | Cl | H | H | H | F | H | F |
| S | Cl | H | H | H | F | H | F |
| O | Cl | H | F | H | H | H | F |
| S | Cl | H | F | H | H | H | F |
| O | Cl | H | H | F | H | H | F |
| S | Cl | H | H | F | H | H | F |
| O | F | H | H | H | H | H | F |
| S | F | H | H | H | H | H | F |
| O | Br | H | H | H | H | H | F |
| S | Br | H | H | H | H | H | F |
| O | F | H | H | H | F | F | Cl |
| S | F | H | H | H | F | F | Cl |
| O | Cl | H | H | H | F | F | Cl |
| S | Cl | H | H | H | F | F | Cl |
| O | Cl | H | H | H | H | F | Cl |
| S | Cl | H | H | H | H | F | Cl |

Verwendet man für die Verfahrensvariante (a) beispielsweise 2-Fluor-4-(1,1,2,2-tetrafluor-ethoxy)-anilin und 2-Fluor-benzoylisothiocyanat als Ausgangsstoffe, so kann der Reaktionsablauf durch das folgende Formelschema wiedergegeben werden:

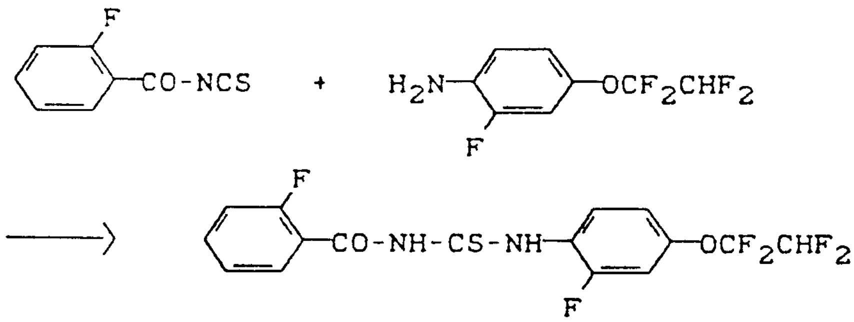

Verwendet man für die Verfahrensvariante (b) beispielsweise 2-Fluor-4-(2-chlor-1,1,2,2-tetrafluor-ethoxy)-phenylisocyanat und 2,4-Difluor-bensoesäureamid als Ausgangsstoffe, so kann der Reaktionsablauf durch das folgende Formelschema wiedergegeben werden;

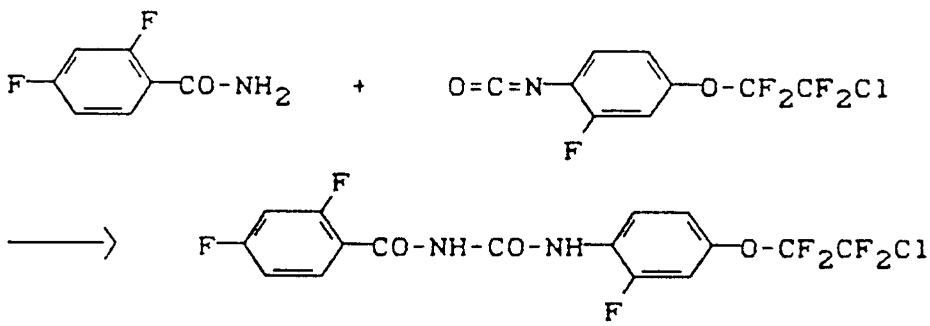

Als Beispiele für die Ausgangsstoffe der Formel (II) seien genannt:
2-Fluor-4-(2-chlor-1,1,2,2-tetrafluor-ethoxy)-anilin, 2-Fluor-4-(2-chlor-1,1,2-trifluor-ethoxy)-anilin und 2-Fluor-4-(1,1,2,3,3,3-hexafluor-propoxy)-anilin.

Man erhält die Verbindungen der Formel (II), wenn man Fluoralkene der allgemeinen Formel (VI),

$F_2C=CF\text{-}R^7$ (VI)

in welcher
$R^7$ die oben angegebene Bedeutung hat,
bzw. Chlorfluoralkane der allgemeinen Formel (VII)

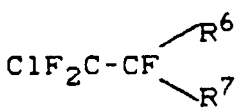

(VII)

in welcher
$R^6$ und $R^7$ die oben angegebenen Bedeutungen haben,
mit 3-Fluor-phenol in Gegenwart einer Base, wie z. B. Natriumhydroxid, und in Gegenwart eines Verdünnungsmittels, wie z. B. Aceton oder Acetonitril, bei Temperaturen zwischen 20 °C und 80 °C umsetzt, die Reaktionsprodukte der allgemeinen Formel (VIII)

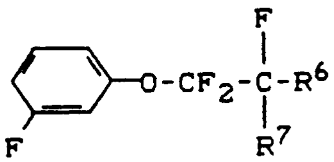 (VIII)

in welcher

$R^6$ und $R^7$ die oben angegebenen Bedeutungen haben,

durch Destillation im Wasserstrahlvakuum isoliert, durch Umsetzung mit Salpetersäure, gegebenenfalls in Gegenwart von Schwefelsäure und gegebenenfalls in Gegenwart eines Verdünnungsmittels, wie z. B. Methylenchlorid, bei Temperaturen zwischen 0 °C und 50 °C nitriert, die Nitrierungsprodukte der allgemeinen Formel (IX)

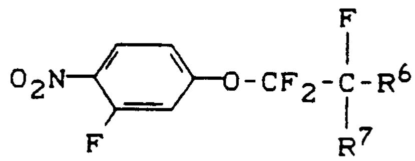 (IX)

in welcher

$R^6$ und $R^7$ die oben angegebenen Bedeutungen haben,

auf übliche Weise, beispielsweise nach Durchmischen der Reaktionslösung mit Eiswasser, Abtrennen der organischen Phase und deren Destillation im Wasserstrahlvakuum, isoliert und nach üblichen Methoden, beispielsweise durch katalytische Hydrierung in Gegenwart von Raney-Nickel und in Gegenwart eines Verdünnungsmittels, wie z. B. Tetrahydrofuran, bei Temperaturen zwischen 0 °C und 80 °C, reduziert.

Als Beispiele für die Ausgangsstoffe der Formel (III) seien genannt:

2-Fluor-, 2-Chlor-, 2-Brom-, 2-Iod-, 2,6-Difluor-, 2,4-Difluor-, 2-Chlor-4-fluor-, 2-Chlor-6-fluor-, 2-Chlor-5-fluor-, 2,3,6-Trichlor-, 2,3,4,5,6-Pentafluor-, 2,4-Dichlor-5-fluor-, 2-Methyl-, 2-Nitro-, 2-Trifluormethyl-, 2,6-Dichlor-, 2,4-Dichlor- und 4-Chlor-benzoylisocyanat bzw. -benzoylisothiocyanat.

Die Ausgangsstoffe der Formel (III) sind bekannt und/oder können nach an sich bekannten Verfahren hergestellt werden (vgl. DE-OS 21 23 236, DE-OS 25 04 982, DE-OS 26 01 780, DE-OS 28 01 316, DE-OS 28 37 086, DE-OS 32 17 619, DE-OS 32 17 620).

Als Beispiele für die Ausgangsstoffe der Formel (IV) seien genannt:

2-Fluor-4-(1,1,2,2-tetrafluor-ethoxy)-phenylisothiocyanat, 2-Fluor-4-(2-chlor-1,1,2,2-tetrafluor-ethoxy)-, 2-Fluor-4-(2-chlor-1,1,2-trifluor-ethoxy)- und 2-Fluor-4-(1,1,2,3,3,3-hexafluor-propoxy)-phenylisocyanat bzw. -phenylisothiocyanat.

Die Ausgangsstoffe der Formel (IV) sind nicht aus der Literatur bekannt und sind Teil der vorliegenden Erfindung. Die Reste Q, $R^6$ und $R^7$ haben die bei den Verbindungen der Formel (I) angegebene allgemeine und bevorzugte Bedeutungen.

Man erhält die Verbindungen der Formel (IV), wenn man entsprechende substituierte Aniline der allgemeinen Formel (II) mit Phosgen bzw. Thiophosgen, gegebenenfalls in Gegenwart eines Verdünnungsmitttels, wie z. B. Toluol, Chlorbenzol, Xylol oder o-Dichlorbenzol, bei Temperaturen zwischen 0 °C und 150 °C umsetzt und das Reaktionsprodukt anschließend unter vermindertem Druck destilliert.

Als Beispiele für die Ausgangsstoffe der Formel (V) seien genannt:

2-Fluor-, 2-Chlor-, 2-Brom-, 2-Iod-, 2,6-Difluor-, 2,4-Difluor-, 2-Chlor-6-fluor-, 2-Chlor-4-fluor-, 2-Chlor-5-fluor-, 2,3,6-Trichlor-, 2,3,4,5,6-Pentafluor-, 2,4-Dichlor-5-fluor-, 2-Methyl-, 2-Nitro-, 2-Trifluormethyl-, 2,6-Dichlor-, 2,4-Dichlor- und 4-Chlor-benzoesäureamid.

Die Ausgangsstoffe der Formel (V) sind bekannt und/oder können nach an sich bekannten Verfahren hergestellt werden (vgl. Literatur zu den Ausgangsstoffen der Formel (III)).

Als Verdünnungsmittel kommen bei der Durchführung der Verfahrensvarianten (a) und (b) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z. B. Acetonitril und Propionitril, Amide, wie z. B. Dimethylformamid, Dimethylacetamid und N-Methyl-pyrrolidon, sowie Tetramethylensulfon.

Als Katalysatoren können für die Umsetzung gemäß Verfahrensvariante (b) vorzugsweise tertiäre Amine, wie Triethylamin und 1,4-Diazabicyclo-[2,2,2]-octan, sowie organische Zinn-Verbindungen, wie z. B. Dibutylzinndilaurat verwendet werden. Der Zusatz solcher Katalysatoren ist jedoch nicht unbedingt erforderlich.

Die Reaktionstemperatur kann bei den Verfahrensvarianten (a) und (b) innerhalb eines größeren Bereiches variiert werden. Im allgemeinen arbeitet man bei der Verfahrensvariante (a) zwischen 20 °C und 180 °C, vorzugsweise zwischen 40 °C und 120 °C und bei der Verfahrensvariante (b) zwischen 20 °C und 200 °C, vorzugsweise zwischen 60 °C und 190 °C. Die erfindungsgemäßen Verfahrensvarianten werden im allgemeinen bei Normaldruck durchgeführt.

Zur Durchführung der erfindungsgemäßen Verfahrensvarianten werden die Ausgangsstoffe gewöhnlich in etwa äquimolaren Mengen eingesetzt. Ein Überschuß der einen oder anderen Reaktionskomponenten bringt keine wesentlichen Vorteile.

Die Aufarbeitung der Reaktionsprodukte geschieht nach üblichen Methoden, z. B. durch Absaugen des ausgefallenen Produktes oder durch Herauslösen von unerwünschten Nebenprodukten aus dem Reaktionsgemisch. Zur Charakterisierung dient der Schmelzpunkt.

Die Verbindungen der allgemeinen Formel (I) eignen sich zur Bekämpfung von tierischen Schädlingen, vorzugsweise Arthropoden, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.,

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp.,

Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in

weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Im vorliegenden Text beziehen sich alle Prozentangaben auf Gewichtsprozente, wenn nichts anderes angegeben wird.

Herstellungsbeispiele

Beispiel 1

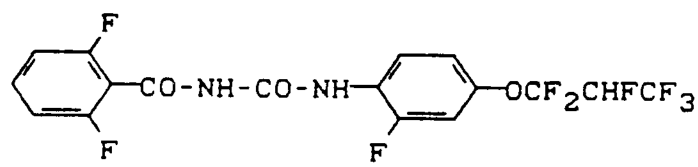

(Verfahrensvariante (a))

1,84 g (0,01 Mol) 2,6-Difluor-benzoylisocyanat werden zu einer auf 60 °C erwärmten Lösung von 2,77 g (0,01 Mol) 2-Fluor-4-(1,1,2,3,3,3-hexafluorpropoxy)-anilin in 60 ml Toluol gegeben und das Reaktionsgemisch wird 30 Minuten bei 80 °C gerührt. Nach Einengen auf etwa das halbe Volumen wird das kristallin angefallene Produkt durch Absaugen isoliert.

Man erhält 4,50 g (98 % der Theorie) 1-(2,6-Difluorbenzoyl)-3-(2-fluor-4-(1,1,2,3,3,3-hexafluor-propoxy)-phenyl)-harnstoff von Schmelzpunkt 164 °C.

Analog Beispiel 1 bzw. entsprechend der allgemeinen Beschreibung der erfindungsgemäßen Verfahrensvarianten (a) und (b) können die in der nachstehenden Tabelle 2 aufgeführten Verbindungen der Formel (I) hergestellt werden.

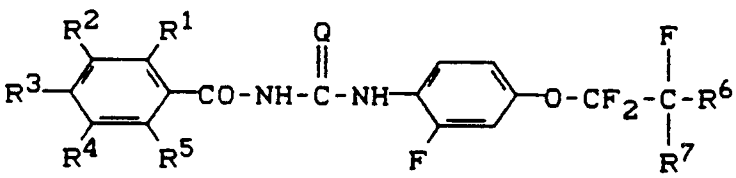

Tabelle 2: Beispiele für die Verbindungen der Formel (I)

| Beisp.-Nr. | Q | $R^1$ | $R^2$ | $R^3$ | $R^4$ | $R^5$ | $R^6$ | $R^7$ | Schmelzpunkt (°C) |
|---|---|---|---|---|---|---|---|---|---|
| 2 | O | Cl | H | F | H | H | H | $CF_3$ | 156 |
| 3 | S | Cl | H | F | H | H | H | $CF_3$ | 179 |
| 4 | S | F | H | H | H | F | H | $CF_3$ | 164 |
| 5 | O | F | H | F | H | H | H | $CF_3$ | 158 |
| 6 | O | Cl | H | H | H | F | H | $CF_3$ | 171 |
| 7 | O | F | F | F | F | F | H | $CF_3$ | 155 |
| 8 | O | Cl | H | Cl | F | H | H | $CF_3$ | 177 |
| 9 | S | Cl | H | H | H | F | H | $CF_3$ | 194 |
| 10 | S | Cl | H | H | H | H | H | $CF_3$ | 155 |
| 11 | O | Cl | H | H | F | H | H | $CF_3$ | 163 |
| 12 | O | F | H | H | H | H | H | $CF_3$ | 163 |
| 13 | O | Cl | H | H | H | H | H | $CF_3$ | 147 |
| 14 | O | Br | H | H | H | H | H | $CF_3$ | 138 |
| 15 | S | F | H | H | H | H | H | $CF_3$ | 65 |
| 16 | S | $CH_3$ | H | H | H | H | H | $CF_3$ | 151 |
| 17 | S | $CF_3$ | H | H | H | H | H | $CF_3$ | 138 |
| 18 | S | H | H | H | H | H | H | $CF_3$ | 79 |
| 19 | O | $NO_2$ | H | H | H | H | H | $CF_3$ | 170 |
| 20 | O | Cl | H | H | H | Cl | H | $CF_3$ | 188 |
| 21 | O | Cl | H | Cl | H | H | H | $CF_3$ | 140 |
| 22 | O | Cl | Cl | H | H | Cl | H | $CF_3$ | 154 |

Tabelle 2 - Fortsetzung

| Beisp.-Nr. | Q | $R^1$ | $R^2$ | $R^3$ | $R^4$ | $R^5$ | $R^6$ | $R^7$ | Schmelz-punkt (°C) |
|---|---|---|---|---|---|---|---|---|---|
| 23 | O | Cl | Cl | H | H | Cl | H | $CF_3$ | 154 |
| 24 | O | H | H | Cl | H | H | H | $CF_3$ | 215 |
| 25 | S | Cl | H | H | H | Cl | H | $CF_3$ | 218 |

Ausgangsstoffe der Formel (II)

Beispiel (II-1)

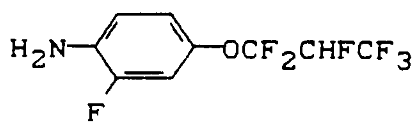

A) 100 g 3-Fluorphenol werden in 300 ml Aceton zusammen mit 20 g Natriumhydroxid bei 55 °C vorgelegt und die Lösung mit Hexafluorpropen gesättigt (Ende der Gasabsorption). Anschließend wird das Aceton zum größten Teil abdestilliert und der Rückstand mit 300 ml Wasser versetzt. Die organische Phase wird abgetrennt und destilliert. Bei einem Siedepunkt von 56 °C - 58 °C/20 mbar ($n_D^{20}$ : 1,3850) fallen 115 g 3-Fluorphenyl-hexafluorpropylether an.

B) In einer Rührapparatur werden 305 g Produkt, wie unter A hergestellt, vorgelegt und 400 ml trockenes Dichlormethan zugegeben. Dann tropft man 110 g Nitriersäure (332 g Salpetersäure und 67 g Schwefelsäure) bei 20 °C zu, rührt für eine Stunde bei dieser Temperatur und anschließend für eine Stunde bei 40 °C nach. Die abgekühlte Reaktionsmischung wird auf Eis gegossen und dann nach gründlichem Durchmischen die organische Phase abgetrennt. Fraktionierte Destillation liefert bei einem Siedepunkt von 92 °C - 95 °C/10 mbar ($n_D^{20}$ : 1,4270) 135 g (2-Nitro-5-fluor-phenyl)-hexafluorpropylether, einen Zwischenlauf von 43 g und bei einem Siedepunkt von 104 °C - 105 °C/10 mbar ($n_D^{20}$ : 1,4325) 87 g (4-Nitro-3-fluor-phenyl)-hexafluorpropylether.

C) In einer Hydrierapparatur werden 87 g (4-Nitro-3-fluor-phenyl)-hexafluor-propylether in 380 ml Tetrahydrofuran und mit 10 g Raney-Nickel vorgelegt und bei 25 °C - 45 °C mit 30 bar Wasserstoff hydriert, bis kein Wasserstoff mehr verbraucht wird. Nach Entspannen des Autoklaven wird die Lösung filtriert und das Filtrat destilliert. Bei dem Siedebereich von 105 °C - 107 °C/20 mbar gehen 59 g 2-Fluor-4-hexafluorpropoxy-anilin ($n_D^{20}$ : 1,4230) obiger Strukturformel über.

Im vorstehenden Text sind "hexafluorpropyl" bzw. "hexafluorpropoxy" jeweils in 1,1,2,3,3,3-Stellung durch Fluor substituiert.

Analog erhält man

(II-2) 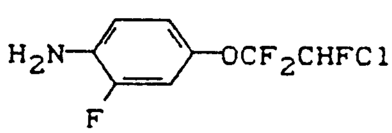

Ausgangsstoffe der Formel (IV)

Beispiel (IV-1)

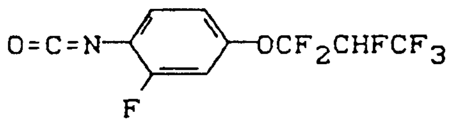

300 ml trockenes Toluol werden auf 0 - 5 °C abgekühlt und bei dieser Temperatur 20 g Phosgen eingegast. Zu dieser Phosgenlösung tropft man bei 0 - 5 °C eine Lösung von 55,6 g (0,2 Mol) 2-Fluor-4-(1,1,2,3,3,3-hexafluorpropoxy)-anilin in 50 ml trockenem Toluol. Anschließend wird der Ansatz unter gleichzeitigem Einleiten von Phosgen zum Sieden erhitzt und 3 Stunden bei Siedetemperatur gehalten. Das überschüssige Phosgen wird mit Stickstoff ausgeblasen und das Lösungsmittel im Vakuum abdestilliert. Der Rückstand wird destilliert.

Man erhält 46 g (75,5 % der Theorie) 2-Fluor-4-(1,1,2,3,3,3-hexafluor-propoxy)-phenylisocyanat vom Siedepunkt $Kp_2$ = 72 °C, $n_D^{20}$ : 1,4258.

Analog erhält man

(IV-2) 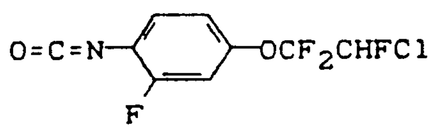

Verwendungsbeispiele

Im folgenden Verwendungsbeispiel wird folgende Verbindung als Vergleichssubstanz eingesetzt:

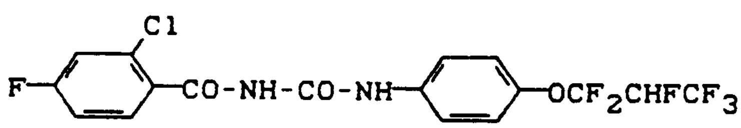 (A)

(bekannt aus DE-OS 32 17 619).

Beispiel A

Spodoptera-Test

Lösungsmittel: 15 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z. B. bei einer beispielhaften Konzentration von 0,00001 % nach 7 Tagen die Verbindungen der Herstellungsbeispiele 1, 2, 4, 6, 9, 10, 12, 13 und 14 eine 100 %ige, die Verbindung aus Beispiel 3 eine 80 %ige und die Verbindung aus Beispiel 11 eine 90 %ige Abtötung, während die Vergleichsverbindung (A) keine Abtötung (0 %) ergab.

**Patentansprüche**

1. Substituierte Benzoyl(thio)harnstoffe der allgemeinen Formel (I),

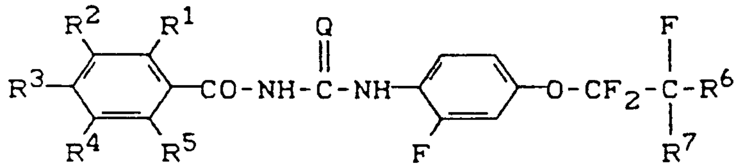

(I)

in welcher
- Q für Sauerstoff oder Schwefel steht,
- $R^1$ für Wasserstoff, Halogen, Nitro, Methyl oder Trifluormethyl steht,
- $R^2$ für Wasserstoff, Fluor oder Chlor steht,
- $R^3$ für Wasserstoff, Fluor oder Chlor steht,
- $R^4$ für Wasserstoff oder Fluor steht,
- $R^5$ für Wasserstoff oder Halogen steht,
- $R^6$ für Wasserstoff, Chlor oder Fluor steht und
- $R^7$ für Fluor, Chlor oder Trifluormethyl steht,

wobei die Restekombination ausgenommen ist, in welcher $R^1$, $R^5$ und $R^7$ für Fluor, $R^2$, $R^3$, $R^4$ und $R^6$ für Wasserstoff und Q für Sauerstoff stehen.

2. Verbindungen gemäß Anspruch 1, wobei
- Q für Sauerstoff oder Schwefel steht,
- $R^1$ für Wasserstoff, Fluor, Chlor, Brom, Nitro, Methyl oder Trifluormethyl steht,
- $R^2$ für Wasserstoff, Fluor oder Chlor steht,
- $R^3$ für Wasserstoff, Fluor oder Chlor steht,
- $R^4$ für Wasserstoff oder Fluor steht,
- $R^5$ für Wasserstoff, Fluor oder Chlor steht,
- $R^6$ für Wasserstoff, Chlor oder Fluor steht und
- $R^7$ für Fluor, Chlor oder Trifluormethyl steht,

wobei die Restekombination ausgenommen ist, in welcher $R^1$, $R^5$ und $R^7$ für Fluor, $R^2$, $R^3$, $R^4$ und $R^6$ für Wasserstoff und Q für Sauerstoff stehen.

3. Verbindungen gemäß Anspruch 1, wobei
- Q für Sauerstoff oder Schwefel steht,
- $R^1$ für Fluor, Chlor oder Brom steht,
- $R^2$ für Wasserstoff steht,
- $R^3$ für Wasserstoff oder Fluor steht,
- $R^4$ für Wasserstoff oder Fluor steht,
- $R^5$ für Wasserstoff oder Fluor steht,
- $R^6$ für Wasserstoff steht und
- $R^7$ für Trifluormethyl steht.

4. Verfahren zur Herstellung der substituierten Benzoyl(thio)harnstoffe der allgemeinen Formel (I),

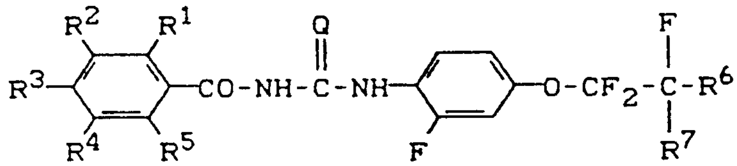

(I)

in welcher
- Q für Sauerstoff oder Schwefel steht,
- $R^1$ für Wasserstoff, Halogen, Nitro, Methyl oder Trifluormethyl steht,

$R^2$ für Wasserstoff, Fluor oder Chlor steht,
$R^3$ für Wasserstoff, Fluor oder Chlor steht,
$R^4$ für Wasserstoff oder Fluor steht,
$R^5$ für Wasserstoff oder Halogen steht,
$R^6$ für Wasserstoff, Chlor oder Fluor steht und
$R^7$ für Fluor, Chlor oder Trifluormethyl steht,

wobei die Restekombination ausgenommen ist, in welcher $R^1$, $R^5$ und $R^7$ für Fluor, $R^2$, $R^3$, $R^4$ und $R^6$ für Wasserstoff und Q für Sauerstoff stehen.

dadurch gekennzeichnet, daß man

(a) substituierte Aniline der allgemeinen Formel (II),

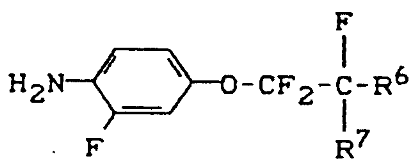

(II)

in welcher

$R^6$ und $R^7$ die oben angegebenen Bedeutungen haben,

mit Benzoyliso(thio)cyanaten der allgemeinen Formel (III)

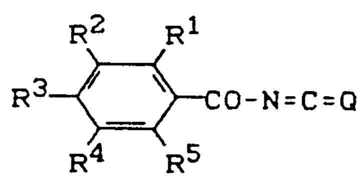

(III)

in welcher

Q, $R^1$, $R^2$, $R^3$, $R^4$ und $R^5$ die oben angegebenen Bedeutungen haben,

gegebenenfalls in Gegenwart eines Verdünnungsmittels umsetzt, oder

(b) substituierte Phenyliso(thio)cyanate der allgemeinen Formel (IV)

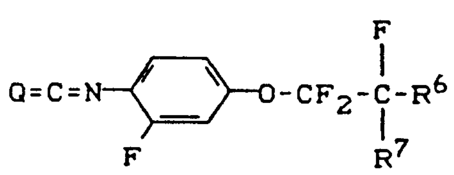

(IV)

in welcher

Q, $R^6$ und $R^7$ die oben angegebenen Bedeutungen haben,

mit Benzoesäureamiden der allgemeinen Formel (V)

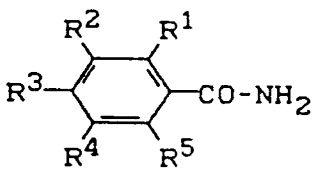

(V)

in welcher

$R^1$, $R^2$, $R^3$, $R^4$ und $R^5$ die oben angegebenen Bedeutungen haben,

gegebenenfalls in Gegenwart eines Katalysators und gegebenenfalls in Gegenwart eines Verdünnungsmittels umsetzt.

**5.** Schädlingsbekämpfungsmittel, gekennzeichnet durch einen Gehalt an mindestens einer Verbindung der Formel (I), gemäß den Ansprüchen 1 oder 4.

**6.** Verwendung von Verbindungen der Formel (I) gemäß den Ansprüchen 1 oder 4 zur Bekämpfung von Schädlingen.

**7.** Verfahren zur Bekämpfung von Schädlingen, dadurch gekennzeichnet, daß man Verbindungen der Formel (I) gemäß den Ansprüchen 1 oder 4 auf Schädlinge und/oder ihren Lebensraum einwirken läßt.

**8.** Verfahren zur Herstellung von Schädlingsbekämpfungsmitteln, dadurch gekennzeichnet, daß man Verbindungen der Formel (I) gemäß den Ansprüchen 1 oder 4 mit Streckmitteln und/oder oberflächenaktiven Mitteln vermischt.

**9.** Substituierte Phenyliso(thio)cyanate der allgemeinen Formel (IV)

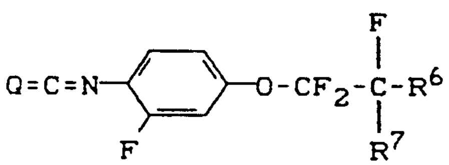

(IV)

in welcher

Q für Sauerstoff oder Schwefel steht,

$R^6$ für Wasserstoff, Chlor oder Fluor steht und

$R^7$ für Fluor, Chlor oder Trifluormethyl steht,

wobei die Restekombination ausgenommen ist, in welcher $R^6$ für Wasserstoff, $R^7$ für Fluor und Q für Sauerstoff stehen.

**10.** Verfahren zur Herstellung der Verbindungen gemäß Anspruch 9, dadurch gekennzeichnet, daß man die Verbindungen der Formel (II)

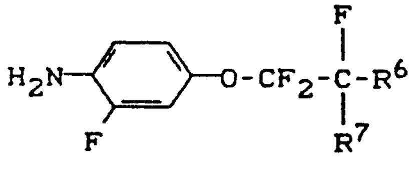

(II)

in welcher

$R^6$ und $R^7$ die in Anspruch 9 angegebene Bedeutung haben,

mit Phosgen bzw. Thiophosgen, gegebenenfalls in Gegenwart eines Verdünnungsmittels bei Temperaturen zwischen 0 ° C und 150 ° C umsetzt.

**Claims**

**1.** Substituted benzoyl(thio)ureas of the general formula (I)

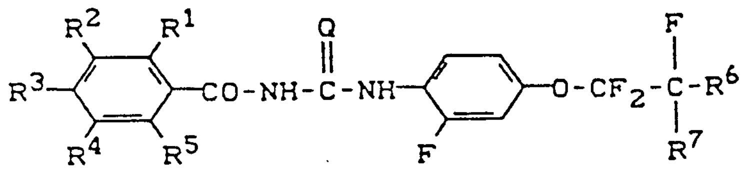

(I)

in which

Q stands for oxygen or sulphur,
$R^1$ stands for hydrogen, halogen, nitro, methyl or trifluoromethyl,
$R^2$ stands for hydrogen, fluorine or chlorine,
$R^3$ stands for hydrogen, fluorine or chlorine,
$R^4$ stands for hydrogen or fluorine,
$R^5$ stands for hydrogen or halogen,
$R^6$ stands for hydrogen, chlorine or fluorine and
$R^7$ stands for fluorine, chlorine or trifluoromethyl,

with the exception of the combination of radicals in which $R^1$, $R^5$ and $R^7$ stand for fluorine, $R^2$, $R^3$, $R^4$ and $R^6$ stand for hydrogen and Q stands for oxygen.

**2.** Compounds according to Claim 1, where

Q stands for oxygen or sulphur,
$R^1$ stands for hydrogen, fluorine, chlorine, bromine, nitro, methyl or trifluoromethyl,
$R^2$ stands for hydrogen, fluorine or chlorine,
$R^3$ stands for hydrogen, fluorine or chlorine,
$R^4$ stands for hydrogen or fluorine,
$R^5$ stands for hydrogen, fluorine or chlorine,
$R^6$ stands for hydrogen, chlorine or fluorine and
$R^7$ stands for fluorine, chlorine or trifluoromethyl,

with the exception of the combination of radicals in which $R^1$, $R^5$ and $R^7$ stand for fluorine, $R^2$, $R^3$, $R^4$ and $R^6$ stand for hydrogen and Q stands for oxygen.

**3.** Compounds according to Claim 1, where

Q stands for oxygen or sulphur,
$R^1$ stands for fluorine, chlorine or bromine,
$R^2$ stands for hydrogen,
$R^3$ stands for hydrogen or fluorine,
$R^4$ stands for hydrogen or fluorine,
$R^5$ stands for hydrogen or fluorine,
$R^6$ stands for hydrogen and
$R^7$ stands for trifluoromethyl, with the exception of

the combination of radicals in which $R^1$, $R^5$ and $R^7$ stand for fluorine, $R^2$, $R^3$, $R^4$ and $R^6$ stand for hydrogen and Q stands for oxygen.

**4.** Process for the preparation of the substituted benzoyl(thio)ureas of the general formula (I)

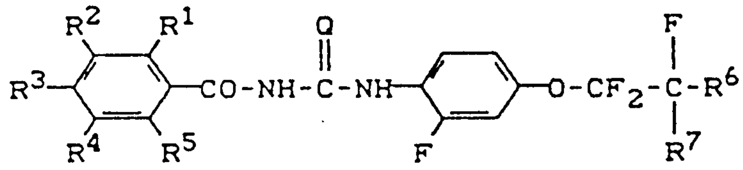

(I)

in which

Q stands for oxygen or sulphur,
$R^1$ stands for hydrogen, halogen, nitro, methyl or trifluoromethyl,
$R^2$ stands for hydrogen, fluorine or chlorine,
$R^3$ stands for hydrogen, fluorine or chlorine,
$R^4$ stands for hydrogen or fluorine,
$R^5$ stands for hydrogen or halogen,
$R^6$ stands for hydrogen, chlorine or fluorine and
$R^7$ stands for fluorine, chlorine or trifluoromethyl,

with the exception of the combination of radicals in which $R^1$, $R^5$ and $R^7$ stand for fluorine, $R^2$, $R^3$, $R^4$ and $R^6$ stand for hydrogen and Q stands for oxygen, characterised in that

(a) substituted anilines of the general formula (II)

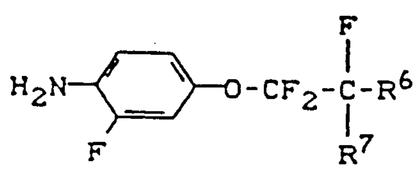

(II)

in which
$R^6$ and $R^7$ have the abovementioned meanings,
are reacted with benzoyl iso(thio)cyanates of the general formula (III)

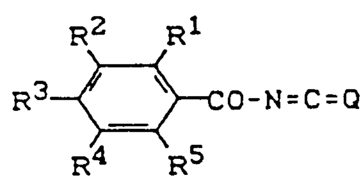

(III)

in which
Q, $R^1$, $R^2$, $R^3$, $R^4$ and $R^5$ have the abovementioned meanings,
if appropriate in the presence of a diluent, or

(b) substituted phenyl iso(thio)cyanates of the general formula (IV)

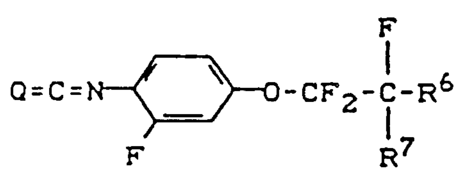

(IV)

in which
Q, $R^6$ and $R^7$ have the abovementioned meanings,
are reacted with benzamides of the general formula (V)

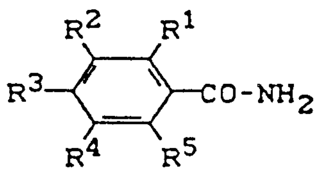

(V)

in which

$R^1$, $R^2$, $R^3$, $R^4$ and $R^5$ have the abovementioned meanings,

if appropriate in the presence of a catalyst and if appropriate in the presence of a diluent.

**5.** Pesticides, characterised in that they contain at least one compound of the formula (I) according to Claims 1 or 4.

**6.** Use of compounds of the formula (I) according to Claims 1 or 4 for combating pests.

**7.** Process for combating pests, characterised in that compounds of the formula (I) according to Claims 1 or 4 are allowed to act on pests and/or their environment.

**8.** Process for the preparation of pesticides, characterised in that compounds of the formula (I) according to Claims 1 or 4 are mixed with extenders and/or surface-active agents.

**9.** Substituted phenyl iso(thio)cyanates of the general formula (IV)

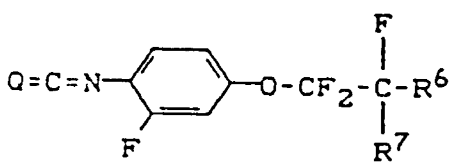

(IV)

in which

Q stands for oxygen or sulphur,

$R^6$ stands for hydrogen, chlorine or fluorine and

$R^7$ stands for fluorine, chlorine or trifluoromethyl,

with the exception of the combination of radicals in which $R^6$ stands for hydrogen, $R^7$ stands for fluorine and Q stands for oxygen.

**10.** Process for the preparation of the compounds according to Claim 9, characterised in that the compounds of the formula (II)

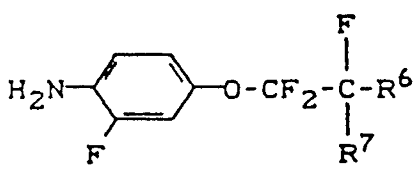

(II)

in which

$R^6$ and $R^7$ have the meaning indicated in Claim 9, are reacted with phosgene or thiophosgene, if appropriate in the presence of a diluent, at temperatures of between 0 ° C and 150 ° C.

## Revendications

**1.** Benzoyl(thio)urées substituées de formule générale (I),

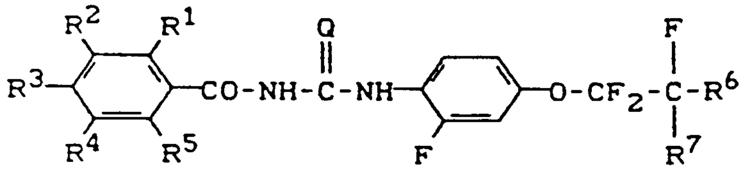

(I)

dans laquelle

Q représente l'oxygène ou le soufre,

$R^1$ est l'hydrogène, un halogène, un groupe nitro, méthyle ou trifluorométhyle,

$R^2$ est l'hydrogène, le fluor ou le chlore,

$R^3$ est l'hydrogène, le fluor ou le chlore,

$R^4$ est l'hydrogène ou le fluor,

$R^5$ est l'hydrogène ou un halogène,

$R^6$ est l'hydrogène, le chlore ou le fluor, et

$R^7$ est le fluor, le chlore ou un groupe trifluorométhyle,

la combinaison de restes selon laquelle $R^1$, $R^5$ et $R^7$ représentent le fluor, $R^2$, $R^3$, $R^4$ et $R^6$ représentent l'hydrogène et Q représente l'oxygène étant exclue.

**2.** Composés suivant la revendication 1, dans lesquels

Q représente l'oxygène ou le soufre,

$R^1$ est l'hydrogène, le fluor, le chlore, le brome, un groupe nitro, méthyle ou trifluorométhyle,

$R^2$ représente l'hydrogène, le fluor ou le chlore,

$R^3$ représente l'hydrogène, le fluor ou le chlore,

$R^4$ représente l'hydrogène ou le fluor,

$R^5$ représente l'hydrogène, le fluor ou le chlore,

$R^6$ représente l'hydrogène, le chlore ou le fluor, et

$R^7$ représente le fluor, le chlore ou un groupe trifluorométhyle,

la combinaison de restes selon laquelle $R^1$, $R^5$ et $R^7$ représentent le fluor, $R^2$, $R^3$, $R^4$ et $R^6$ représentent l'hydrogène et Q représente l'oxygène étant exclue.

**3.** Composés suivant la revendication 1, dans lesquels

Q représente l'oxygène ou le soufre,

$R^1$ est le fluor, le chlore ou le brome,

$R^2$ est l'hydrogène,

$R^3$ est l'hydrogène ou le fluor,

$R^4$ est l'hydrogène ou le fluor,

$R^5$ est l'hydrogène ou le fluor,

$R^6$ est l'hydrogène, et

$R^7$ est un groupe trifluorométhyle.

**4.** Procédé de production de benzoyl(thio)urées substituées de formule générale (I),

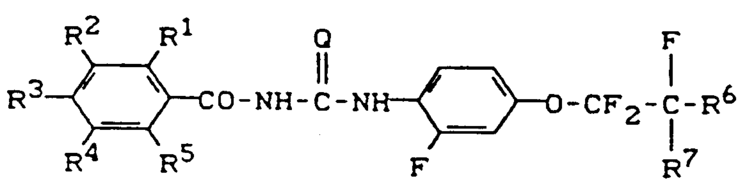

(I)

dans laquelle

Q est l'oxygène ou le soufre,

$R^1$ est l'hydrogène, un halogène, un groupe nitro, méthyle ou trifluorométhyle,

$R^2$ est l'hydrogène, le fluor ou le chlore,

$R^3$ est l'hydrogène, le fluor ou le chlore,

$R^4$ est l'hydrogène ou le fluor,

$R^5$ est l'hydrogène ou un halogène,
$R^6$ est l'hydrogène, le chlore ou le fluor, et
$R^7$ est le fluor, le chlore ou un groupe trifluorométhyle,

la combinaison de restes selon laquelle $R^1$, $R^5$ et $R^7$ représentent le fluor, $R^2$, $R^3$, $R^4$ et $R^6$ représentent l'hydrogène et Q représente l'oxygène étant exclue,

caractérisé en ce que

(a) on fait réagir des anilines substituées de formule générale (II),

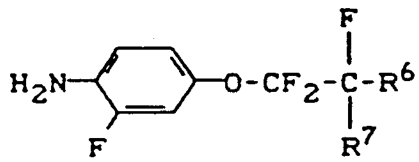

(II)

dans laquelle
$R^6$ et $R^7$ ont les définitions indiquées ci-dessus,
avec des iso(thio)cyanates de benzoyle de formule générale (III)

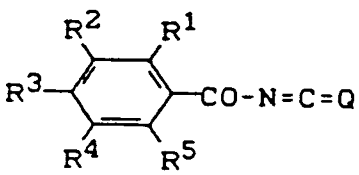

(III)

dans laquelle
Q, $R^1$, $R^2$, $R^3$, $R^4$ et $R^5$ ont les définitions indiquées ci-dessus,
éventuellement en présence d'un diluant, ou bien

(b) on fait réagir des iso(thio)cyanates de phényle substitués de formule générale (IV)

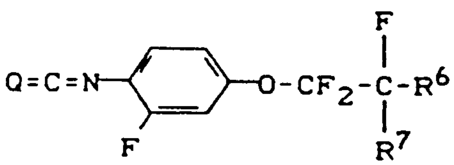

(IV)

dans laquelle
Q, $R^6$ et $R^7$ ont les définitions indiquées ci-dessus, avec des amides d'acide benzoïque de formule générale (V)

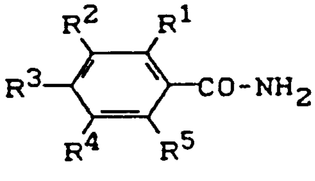

(V)

dans laquelle
$R^1$, $R^2$, $R^3$, $R^4$ et $R^5$ ont les définitions indiquées ci-dessus, éventuellement en présence d'un catalyseur et, le cas échéant, en présence d'un diluant.

**5.** Compositions pesticides, caractérisées par une teneur en au moins un composé de formule (I) suivant les revendications 1 ou 4.

6. Utilisation de composés de formule (I) suivant les revendications 1 ou 4 pour combattre des parasites.

7. Procédé pour combattre des parasites, caractérisé en ce qu'on fait agir des composés de formule (I) suivant les revendications 1 ou 4 sur les parasites et/ou sur leur milieu.

8. Procédé de préparation de compositions pesticides, caractérisé en ce qu'on mélange des composés de formule (I) suivant les revendications 1 ou 4 avec des diluants et/ou des agents tensio-actifs.

9. Iso(thio)cyanates substitués de formule générale (IV)

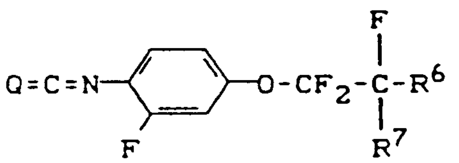

(IV)

dans laquelle

Q est l'oxygène ou le soufre,
$R^6$ est l'hydrogène, le chlore ou le fluor et
$R^7$ est le fluor, le chlore ou un groupe trifluorométhyle,

la combinaison de restes selon laquelle $R^6$ est l'hydrogène, $R^7$ est le fluor et Q est l'oxygène étant exclue.

10. Procédé de préparation de composés suivant la revendication 9, caractérisé en ce qu'on fait réagir les composés de formule (II)

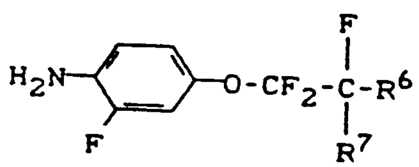

(II)

dans laquelle

$R^6$ et $R^7$ ont la définition indiquée dans la revendication 9,

avec le phosgène ou le thiophosgène, éventuellement en présence d'un diluant, à des températures comprises entre 0 ° C et 150 ° C.